# EUROPEAN PATENT APPLICATION

(11) **EP 4 618 098 A1**
(43) Date of publication of application: **17.09.2025**
(21) Application number: 24163643.0
(22) Date of filing: 14.03.2024
(51) Int. Cl.: G16H 40/63, G16H 40/67, G16H 50/30, G16H 50/20

(54) **A SYSTEM FOR REMOTE MONITORING A PATIENT WITH PARKINSON'S DISEASE**

(71) Applicant: Nordicinfu Care AB, 167 51 Bromma (SE); L'AIR LIQUIDE, SOCIETE ANONYME POUR L'ETUDE ET L'EXPLOITATION DES PROCEDES GEORGES CLAUDE, 75007 Paris (FR)
(72) Inventor: JAUNATRE, Alexandre, 167 51 Bromma (SE); RUDILLA, David, 28020 Madrid (ES)
(74) Representative: Air Liquide

(57) **Abstract**

The invention concerns a system for remote monitoring a patient (P) with Parkinson's disease comprising sensor means (2) for measuring a motor status of the patient (P) over time and communication means (3) for providing the motor status measurements to remote server means (4) configured for running softwares for processing the motor status measurements and determining different symptoms related to the Parkinson's disease of the patient, i.e. dyskinesia, "OFF" time and freezing of gait, and further for evaluating a respective degree of severity of a plurality of symptoms and evaluating a general state of the patient based on the degrees of severity determined. Display means (5.1, 5.2) are provided for displaying a graphical representation (6) of the different symptoms that have been determined and their degree of severity, and/or the general state of the patient.

## Description

The present invention concerns a system for remote monitoring a patient with Parkinson's disease (PD), i.e. a remote monitoring system, useable as a decision making support tool for classifying patients according to their symptom severity and delivering personalized care plan, that can be part of a decision making support tool or system useable for improving the treatments of the patients and, when needed, for providing them a dedicated care support.

Parkinson's disease is a chronic degenerative disorder that progressively affects the nervous central system and the parts of the body controlled by the nerves. Symptoms appear slowly and commonly include noticeable tremor, stiffness, slowing of movement, no arm-swing while walking, speech issues... Parkinson's disease symptoms usually worsen as the disease progresses over time.

The disease cannot be cured but some medications or drugs, such as apomorphine, L-Dopa, Levodopa, Carbidopa or similar, can significantly improve the symptoms and thus the quality of life of the patients.

As the state of the patient usually negatively evolves over time, it is crucial to be able to properly monitor the patient in order to determine his/her state of health and to provide the right intervention accordingly. However, for obvious reasons, it is not possible that a dedicated nurse is assigned to only one patient on a full-time basis. Therefore, some documents propose remote system for controlling the dose of medication to be administrated to the patient.

Thus, EP3846173 teaches a remote titration system, i.e. dose modification system, of a patient suffering from Parkinson's disease comprising input means for inputting different doses of an anti parkinsonian medication to be administered, daily administration times of said doses and further evaluations of a prefixed efficacy criterion for said drug doses. Various information can be displayed. Further, telecommunications means allow exchanging data.

Further, EP3783617 teaches a monitoring system of a patient suffering from Parkinson's disease comprising a perfusion pump that is remotely controlled by pilot means on the basis of a measured flowrate that can be modified, if need be, by a physician or the like.

However, those documents are limited to a remote control of the dosage of the medication to be administrated to patients suffering from Parkinson's disease.

In other words, a problem is that it is still difficult to remotely monitor a patient suffering from Parkinson's disease for determining his/her state of health over time and, if required, for proactively taking appropriate measures, such as adapting his/her medication and/or sending a nurse to the patient's home, when a deterioration or a change of his/her state of health is detected, in particular a change of one or several symptoms of the disease affecting the patient.

Further, neurologists are generally not able to properly classify their patients, i.e. determine the degree of severity of their disease over the time, as most of the patients meet with their neurologist only once a year, which is below some national health recommendations that advocate several evaluations a year.

A goal according to the present invention is to provide an improved system for remote monitoring a patient with Parkinson's disease.

A solution according to the present invention concerns a system for remote monitoring a patient with Parkinson's disease, i.e. a remote monitoring system, useable as a decision making support tool for classifying patients according to their symptom severity and delivering personalized care plan, comprising :
- sensor means configured for measuring a motor status of the patient over time,
- communication means configured for providing at least the motor status measurements provided by said sensor means to remote server means,
- the server means configured for running at least:
   A) a first software configured for :
      - processing at least said motor status measurements and
      - determining different symptoms related to the Parkinson's disease of the patient, said different symptoms being chosen among dyskinesia, "OFF" time and freezing of gait, and
   B) a second software configured for:
      i) evaluating a respective degree of severity of a plurality of said different symptoms and
      ii) evaluating a general state of the patient based on at least the degrees of severity determined for the different symptoms considered in step i),
- and display means configured for displaying a graphical representation comprising at least several of the different symptoms that have been determined by the first software and at least a degree of severity for each of said different symptoms, and/or the general state of the patient.

Depending on the embodiment, a system for remote monitoring a patient according to the present invention can comprise one or several of the following additional features.

### Sensor means

- the sensor means comprise at least a sensor carried by the patient
- the sensor is arranged on a belt, a watch or any other wearable device.
- the sensor is preferably a Stat-On^{®} sensor or the like.
- the sensor is configured for measuring a motor status of the patient during a period of time of several days or one or several weeks, for instance during one week (i.e. 7 days).
- the motor status comprises patient's motions/movements, motor symptoms related to PD, and severity of said motor symptoms.

### Communication means

- the communication means are configured for providing the motor status measurements and additional patient data or parameters.
- additional patient data or parameters can be provided by a nurse.
- additional patient data or parameters can be recovered by means of PD questionnaires. The questions of said questionnaires can be asked directly to the patient by a nurse visiting the patient at home.
- additional patient data or parameters comprise tremors, gait, functional impact of fluctuations, non-motor symptoms, wearing-off, "5-2-1" (5 L-Dopa dosage + 2 hours of "OFF" time + 1 hour of dyskinesia), 5 L-dopa dosages (number of dosages that are prescribed by the neurologist) and extra-dosages, or any other parameter of interest.
- additional patient data or parameters can comprise two categories (or more) comprising a first category of additional parameters provided by the nurse comprising symtoms (not monitored by the sensor), wearing off, tremors, gait, "5-2-1 ", falls (i.e. loss of balance with collapsing), functional impact of fluctuations.
- additional patient data or parameters can comprise two categories (or more) further comprising a second category of additional parameters corresponding to outcomes comprising Activity Daily Living (ADL), Barthel Index (Independence in daily life), PDQ-8 (Quality of life) and EDQ5D (Global feeling).
- the communication means are configured to communicate using a communication network, such as internet, GSM (4G/5G) or others.
- the communication means comprise a smartphone, a laptop, a digital pad or the like.

### Server means

- the server means comprises one or several servers, such one or several digital platforms or the like.
- the server means comprise one or several (micro)processor(s).
- the (micro)processor(s) runs software(s).
- the server can be or comprise a computer device or the like, i.e. a tool that can be hardware, software or virtual, that offers services to user(s), in particular that processes data or the like.
- the server means comprise a first server comprising or running the first software.
- the server means comprise a second server comprising or running the second software.
- according to an embodiment, the first server cooperates with the second server.
- according to an alternative embodiment, the server means comprise a unique server comprising or running the first and the second softwares.
- the server means are located on a remote site, i.e. not in the patient's home.
- the server means comprise one or several Cloud-type server.
- the severity degree of each symptom can be categorized as being mild, moderate or severe, or other.
- the severity degree is considered as being "mild", when the patient experienced little symptoms and his/her treatment suits him/her without requiring any visit to a hospital.
- the severity degree is considered as being "moderate", when the patient experienced mild symptoms and his/her state of health is deteriorating.
- the severity degree is considered as being "severe", when the patient experienced worrying symptoms and his/her treatment does not suit him/her anymore.
- a specific color can be assigned to each severity degree, for instance green for mild, orange for moderate and red for severe, or any other color.
- further, an additional color can be assigned to patients having a severity degree considered as being "not-improvable", i.e. corresponding to patients having a state of health that cannot be improved or enhanced, for instance a blue color.
- the graphical representation comprises a spider map or similar.
- the general state of the patient comprises a general degree of severity calculated from the different degrees of severity for the different symptoms.
- the different degrees of severity for the different symptoms are preferably pondered.

### Display means

- the display means comprise at least one display screen or the like.
- the display means are or comprise at least one color display screen.
- the display means are or comprise at least one laptop, computer, smartphone, digital tablet (e.g. I-Pad or similar)
- the display means comprises a first display screen located in a hospital, such as a neurology hospital or the like.
- the first display screen is embedded in a laptop, a computer or the like.
- the display means comprises a second display screen located at the patient's home.
- the second display screen is embedded in a smartphone, a digital tablet (e.g. I-Pad or similar) or the like.
- the display means provide or display a graphical representation comprising different symptoms that have been determined by the first software, a degree of severity for each of said different symptoms.
- the display means provide or display a graphical representation comprising different symptoms that have been determined by the first software, a degree of severity for each of said different symptoms, and the general state of the patient.
- the display means display the graphical representation comprising a curve (i.e. line) or similar linking a plurality of dots or similar representing the degrees of severity for the different symptoms shown in the graphical representation.
- the graphical representation displayed by the display means comprises at least one curve linking a plurality of dots representing the degrees of severity of the different symptoms.
- the graphical representation comprises degrees of severity rated from 0 to 3.
- the graphical representation comprises the degrees of severity flagged by specific colors, such as green, orange and red.
- the graphical representation comprises the general state of the patient flagged by a color, such as green, orange and red.
- the graphical representation comprises the spider map and said at least one curve linking the plurality of dots representing the degrees of severity of the different symptoms.

### Definitions

In relation to the present invention :
- the abbreviation "PD" stands for Parkinson's disease.
- the terms "medication" and "drug" are considered as equivalent,
- the terms "dose", "dosage" and "concentration" are considered as equivalent,
- the term "means" are considered equivalent and substitutable by the term "device". For instance, the terms "sensor means" can be replaced by « sensor device »; the terms "display means" can be replaced by « display device »; the terms "communication means" can be replaced by « communication device », etc....
- a "sensor" is a measurement device that can measure a parameter, a physical quantity or the like, and provide a corresponding measurement value, signal or similar.
- the terms "degree" and "level" are considered as equivalent, in particular the terms "degree of severity" are equivalent to "level of severity".

The invention will now be better understood thanks to the following detailed description, that is illustrative but not limitative, with reference to the enclosed Figures, among which:
- Figure 1 represents an embodiment of a monitoring system according to the present invention.
- Figure 2 represents an example of a graphic representation displayed by the display means of a monitoring system according to the present invention, such the monitoring system shown in Fig. 1.
- Figure 3 shows an example of a care plan that can be established for a patient thanks to the graphic representation displayed by a monitoring system according to the present invention, such as shown in Fig. 2.

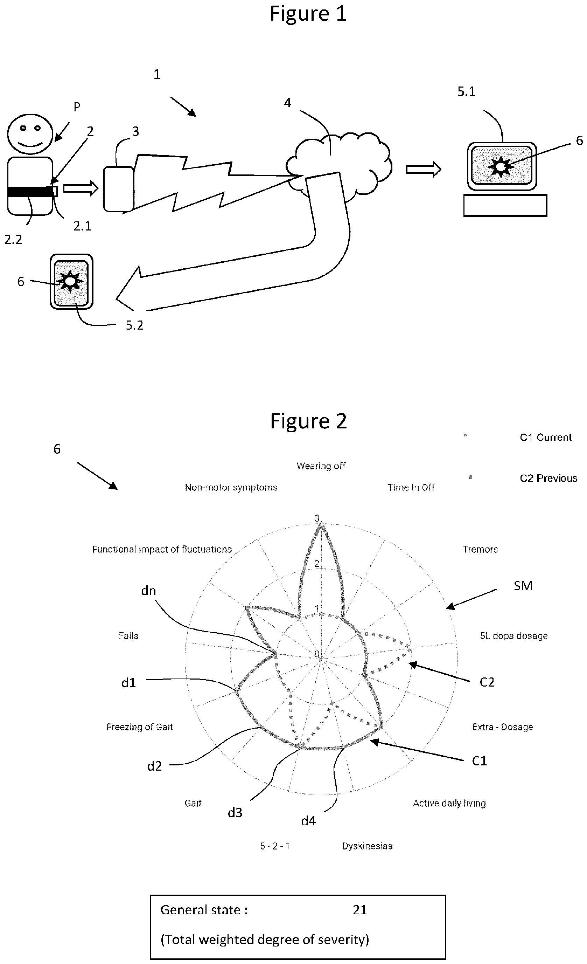

Figure 1 represents an embodiment of a monitoring system 1 according to the present invention for remote monitoring a patient P suffering from Parkinson's disease (PD) and following a treatment related to the disease.

The monitoring system 1 comprises sensor means 2, such as a Stat-on^{®} sensor or any other suitable sensor, that is configured for measuring a motor status of the patient P over time, typically during several days, weeks or months.

In particular, the sensor is configured for providing the following measurements: time awake in hours per day, "OFF" time in hours per day, time with dyskinesia in hours per day, number of freezing of gaits, preferably per day (i.e. 24h).

The motor status measurements made by said sensor means 2 are provided to remote server means 4 thanks to communication means 3, such as a smartphone, a laptop, a digital pad or the like, configured to send the measurements through a suitable communication network, such as GSM (4G/5G), internet/web or the like.

The measurements can be either communicated directly by said sensor means 2 to said communication means 3, for instance by Bluetooth^{®} or the like, or indirectly by a user that inputs the measurements made by said sensor means 2 in the communication means 3.

Preferably, additional data or information related to the patient P, i.e. besides the measurements, can be also transmitted to the server means 4, such as technical information related to the device used in the frame of the treatment, quality or life questionnaires, such as UPDRS (Unified Parkinson Disease Rating Scale) or NMSQ (Non-Motor Symptoms Questionnaire), or others.

The server means 4 can comprise or be an internet platform or the like. They are configured for comprising and/or running at least one software that is configured for processing the motor status measurements received from the communication means 3 as well as, if need be, any other information or data, and determining, i.e. deducing therefrom, different symptoms related to the Parkinson's disease of the patient

Preferably, said server means are configured for running at least two softwares including a first and a second software. The first software is configured for processing motor status measurements provided by said sensor means 2 and deducing therefrom, i.e. determining from said measurement processing, different symptoms related to the Parkinson's disease affecting the patient.

Typically, the different symptoms can be dyskinesia, "OFF" time and freezing of gait. In the frame of the invention:
- "dyskinesia" is an abnormality or impairment of voluntary movement,
- "OFF" time" is when levodopa (PD drug) is no longer working well and symptoms reemerge, such as tremor, slow movement and rigidity,
- "freezing of gait" is a short absence or marked reduction of forward progression of the feet despite the intention to walk of the PD patient.

For instance, for determining the "OFF" time parameter, namely an average time "OFF" per day, first software processes motor status measurements, typically the total of hours "awake" and the total hours "OFF" time of the patient. Then, a "flag" based on defined thresholds, i.e. prefixed and memorized thresholds, or the like is attributed as below explained, for instance a green, orange or red flag.

Similarly, for determining "dyskinesia" parameter, the sensor can provide the total hours "awake" and the total hours with dyskinesia, and for determining "freezing of gait" parameter, the sensor can provide the number of freezing of gait per day, thereby attributing also a "flag" also based on predetermined thresholds or the like, for instance a green, orange or red flag.

In other words, the first software is configured for determining the percentage (%) of time spent in "OFF" per day, and the percentage (%) of time spent with dyskinesia per day and the number of the freezing of gait.

Further, the second software is configured for evaluating a respective degree or level of severity of at least some of said different symptoms and evaluating, i.e. deducing therefrom, a general state of the patient based on the degrees of severity determined for the considered different symptoms and optionally from other parameters, such as questionnaires or the like.

For instance:
- Wearing off can be determined using 19 "double questions". If there is at least one answer that is "Yes -Yes", then the global flag thus attributed to this parameter is "red". Otherwise, it is a "green" flag.
- Tremors are deduced/measured from the tremor highest amplitudes of both arms and legs : no tremors, < 1cm, 1 - 3 cm, 3 - 10 cm and > 10cm. A score can be then established based upon predefined thresholds. Preferably, questions about the impact on patient quality of life (none, mild, moderate, severe) can be used for completing the assessment and thus obtaining a total score. Here again, the total score can be used for determining a flag for tremors according to defined thresholds or the like.
- Similarly, flags can be obtained for 5 L-dopa dosage and extra-dosage from the number of prescribed dosage and predefined thresholds, for 5-2-1 from data related to 5 L-dopa dosage, "OFF" time and dyskinesia, for ADL, gait and falls from questions (5 categories), for instance green, orange or red flags.

The server means 4 can comprise a first server comprising or running the first software, and a second server comprising or running the second software, or according to an alternative embodiment, a unique server comprising or running both softwares.

The server(s) can be or comprise a computer device, i.e. a tool or a system that can be hardware, software and/or virtual, that offers services, in particular that processes data or the like. For instance, the server(s) can comprise be a hardware, i.e. a device, such as a computer or the like, running software(s) thanks to one or several (micro)processors or the like embedded in the hardware. The server(s) can be Cloud servers or any other kind or server.

Furthermore, the system 1 also comprises display means 5.1, 5.2, such as a first display screen 5.1 and a second display screen 5.2, that are configured for displaying a graphical representation 6 representing at least the different symptoms that have been determined by said server means 4 and a categorization of each symptom, i.e. a degree of severity, such as severe, moderate or mild, or similar or others, and preferably the general state of the patient that has been determined.

The general state of the patient can be determined as a value obtained from the degree/level of severity values of the different symptoms that have been considered, preferably pondered values as some symptoms are more important than others.

The degree or level of severity is considered as being :
- Mild, when the patient experienced little symptoms and his/her treatment suits him/her without requiring any visit to a hospital or of a nurse,
- Moderate, when the patient experienced mild symptoms and his/her state of health is deteriorating, and
- Severe, when the patient experienced worrying symptoms and his/her treatment does not suit him/her anymore.

In other words, said display means 5.1, 5.2 are configured for displaying a graphical representation 6 comprising several of the different symptoms that have been determined by the first software and a degree of severity for each of said different symptoms, and preferably the general state of the patient.

Once determined, the severity of each symptom can be displayed in a specific color, i.e. also flagged, for instance green for mild, orange for moderate and red for severe, or any other suitable colors.

Preferably, an additional color, such as blue or another color, can be assigned to a degree of severity considered as being "not-improvable" corresponding to patients having a state of health that cannot be improved or enhanced.

In some embodiments, the general state of the patient is also flagged by such colors, for instance a green, orange or red flag.

The first display screen 5.1 can be located in a care provider's facilities, such as a neurology hospital, whereas the second display screen 5.2 can be located at the patient's home. The display means 5.1, 5.2 can be or comprise the display screens of one or several laptops, computers, smartphones, digital pads or the like.

Of course, the different elements of the system of the invention that require electric current for working properly are powered by the Mains and/or by batteries.

An example of a graphical representation 6 is shown in Fig. 2. In this example, the graphical representation 6, namely a so-called 'spider map' (SM), shows a curve C1 (i.e. a line) linking the dots (d1, d2... dn) or points that represent the current degrees of severity from 0 to 3 for various symptoms including dyskinesia, "OFF time" and freezing of gait, which are the main symptoms that can be determined based on the measurements made by the sensor of the system according to the present invention. Actually, the values from 1 to 3 also represent the colors of the different flags provided, in particular "1" means a 'green flag', "2" means an 'orange flag', and "3" means a 'red flag'.

However, additional symptoms, i.e. secondary symtoms, are also represented in Fig. 2, namely falls, tremors, "5L Dopa dosage, extra dosage, active daily living (ADL), "5-2-1", gait, "OFF" time, non-motor, wearing off.... Said additional symptoms can be determined by the nurse visiting the patient, by means of health related questionnaires or the like, and subsequently provided/transmitted by the nurse to the server means 4 by means of a input device, such as a smartphone, a laptop, an digital tablet or the like.

Once received by the server means 4, they are processed by software(s) as above explained, such as the first and second softwares.

In this example, the general state of the patient is also given as a total score value (here equals to 21), calculated from the various degrees of severity of the different symptoms that have been considered, i.e., the main symptoms, namely dyskinesia, time in/time off and freezing of gait, and the secondary symptoms, namely tremors, falls, 5 L-Dopa dosage, extra dosage, ADL, "5-2-1", gait, "off periods", non-motor, wearing off... as shown in Fig. 2. The values can be weighted, i.e. pondered, values and/or average values, or the like. In other words, in some embodiments, at least some of the degrees of severity of the different symptoms are preferably weighted, for instance extra dosage and non-motor symptoms can have different weights from 1 to 6 (green flag = 1; orange flag = 4; red flag = 6).

The determination of each of the flags is done using predefined thresholds or the like. For instance, regarding the freezing of gait, if the number of freezing of gait per day is from 0 to 5, the flag that is assigned is green, whereas if it is from 6 to 10, the assigned flag is orange, and similarly if it is greater than 10, the assigned flag is red.

Further, the total score value can also be flagged by a specific color depending on the severity as above explained.

Preferably, as shown in Fig.2, an additional curve C2 representing a previous degree of severity is further provided, for instance the degree of severity determined another time, i.e. over a previous period or time, for example from 1 to 6 months before.

Of course, the graphical representation 6, i.e. the "spider map" SM and other information, can be also edited, for instance as a report in PDF format or the like, and further provided to the medical staff.

A report can be edited for each patient P and can comprise the graphical representation 6 and/or other information related to the patient, such as his/her general state that has been determined as above explained, that is useful for the medical staff to assess, confirm or improve the treatment of the PD in the patient.

Thus, thanks to the graphical representation 6, it is possible to detect a need for a visit with a neurologist or a potential change in the treatment provided to the patient, and in response of such a detection, to refer the patient P to a clinic, hospital or the like, such as a neurologic hospital, where a neurologist will be able to modify the treatment if appropriate, for instance using a patient's report.

Indeed, detecting the first changes in symptoms or signs allows preemptive interventions thereby preventing worsening of problems and avoiding complications that would lead to an emergency situation, a hospital admission and unnecessary medical resources/staff.

Thanks to the graphical representation 6, it is also possible to establish, when required or desired, a dedicated care plan to the patient or re-adjust a previous care plan for taking into account variations in the symptoms and their severity.

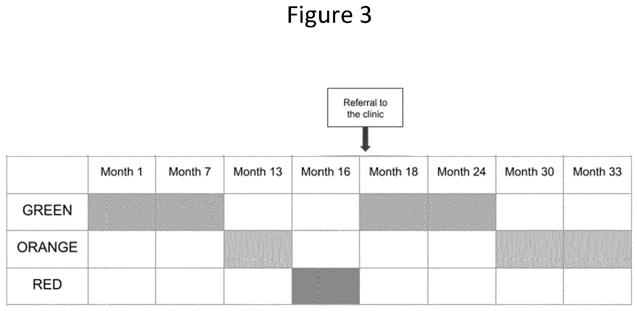

Figure 3 shows an example of such a personalized care plan. It shows that the patient has regular interventions or meetings with a nurse, such as a home visit, a video call or a message exchange (i.e. SMS or the like).

It further provides the date of the next intervention or meeting defined by the global colorized flag, i.e. a given color such as green, orange or red. Thus, the patient knows when he/his has to get the clinic, hospital or the like, namely only when needed, when least one of the symptoms is "red" flagged.

The monitoring system 1 according to the present invention can be used for remotely monitoring patients suffering from PD and as a decision making support tool for classifying patients according to their symptom severity and delivering personalized care plan, thereby improving the treatments of the patients and, when needed, providing them a dedicated care support.

## Claims

1. System (1) for remote monitoring a patient (P) with Parkinson's disease comprising:
- sensor means (2) configured for measuring a motor status of the patient (P) over time,
- communication means (3) configured for providing at least the motor status measurements provided by said sensor means (2) to remote server means (4),
- the server means (4) configured for running at least:
A) a first software configured for :
- processing at least said motor status measurements and
- determining different symptoms related to the Parkinson's disease of the patient, said different symptoms being chosen among dyskinesia, "OFF" time and freezing of gait,
B) a second software configured for:
i) evaluating a respective degree of severity of a plurality of said different symptoms and
ii) evaluating a general state of the patient based on at least the degrees of severity determined for the different symptoms considered in step i),
- and display means (5.1, 5.2) configured for displaying a graphical representation (6) comprising at least several of the different symptoms that have been determined by the first software and a degree of severity for each of said different symptoms, and/or the general state of the patient.

2. System according to Claim 1, **characterized in that** the sensor means (2) comprise at least a sensor (2.1) carried by the patient, preferably a sensor (2.1) arranged on a belt (2.2).

3. System according to Claim 1, **characterized in that** the server means comprise at least a first server comprising or running the first software, and a second server comprising or running the second software.

4. System according to Claim 1, **characterized in that** the general state of the patient comprises a general degree of severity calculated from the different degrees of severity for the different symptoms, preferably weighted degrees of severity.

5. System according to Claim 1, **characterized in that** the graphical representation (6) comprises a spider map (SM).

6. System according to Claim 1, **characterized in that** the display means comprise at least one laptop, computer, smartphone and digital tablet.

7. System according to any one of Claims 1 or 6, **characterized in that** the display means (5.1, 5.2) comprise at least one first display screen located in a neurology hospital and/or a second display screen located at the patient's home.

8. System according to Claim 1, **characterized in that** the communication means (3) are configured to communicate using a communication network chosen among internet and GSM (4G/5G).

9. System according to Claim to any one of Claims 1 or 8, **characterized in that** the communication means (3) comprise a smartphone, a laptop or a digital pad.

10. System according to Claim 1, **characterized in that** the degrees of severity are chosen among severe, moderate and mild.

11. System according to Claim 1, **characterized in that** the graphical representation (6) comprises degrees of severity rated from 0 to 3.

12. System according to Claim 1, **characterized in that** the graphical representation (6) comprises the degrees of severity flagged by specific colors.

13. System according to Claim 1, **characterized in that** the graphical representation (6) comprises the general state of the patient flagged by a color.

14. System according to Claim 1, **characterized in that** the graphical representation (6) displayed by the display means (5.1, 5.2) comprises at least one curve (C1) linking a plurality of dots (d1, d2... dn) representing the degrees of severity of the different symptoms.

15. System according to Claims 5 and 14, **characterized in that** the graphical representation (6) comprises the spider map (SM) and said at least one curve (C1) linking the plurality of dots (d1, d2... dn) representing the degrees of severity of the different symptoms.

## Amended claims

### Amended claims in accordance with Rule 137(2) EPC.

1. System (1) for remote monitoring a patient (P) with Parkinson's disease comprising:
- sensor means (2) configured for measuring a motor status of the patient (P) over time,
- communication means (3) configured for providing at least the motor status measurements provided by said sensor means (2) to remote server means (4),
- the server means (4) configured for running at least :
A) a first software configured for :
- processing at least said motor status measurements and
- determining different symptoms related to the Parkinson's disease of the patient, said different symptoms being chosen among dyskinesia, "OFF" time and freezing of gait,
B) a second software configured for :
i) evaluating a respective degree of severity of a plurality of said different symptoms and
ii) evaluating a general state of the patient based on at least the degrees of severity determined for the different symptoms considered in step i),
- and display means (5.1, 5.2) configured for displaying a graphical representation (6) comprising at least several of the different symptoms that have been determined by the first software and a degree of severity for each of said different symptoms, wherein the graphical representation (6) comprises a spider map (SM) comprising at least one curve (C1) linking a plurality of dots (d1, d2... dn) representing the degrees of severity of the different symptoms.

2. System according to Claim 1, **characterized in that** the sensor means (2) comprise at least a sensor (2.1) carried by the patient, preferably a sensor (2.1) arranged on a belt (2.2).

3. System according to Claim 1, **characterized in that** the server means comprise at least a first server comprising or running the first software, and a second server comprising or running the second software.

4. System according to Claim 1, **characterized in that** the general state of the patient comprises a general degree of severity calculated from the different degrees of severity for the different symptoms, preferably weighted degrees of severity.

5. System according to Claim 1, **characterized in that** the graphical representation (6) further comprises the general state of the patient.

6. System according to Claim 1, **characterized in that** the display means comprise at least one laptop, computer, smartphone and digital tablet.

7. System according to any one of Claims 1 or 6, **characterized in that** the display means (5.1, 5.2) comprise at least one first display screen located in a neurology hospital and/or a second display screen located at the patient's home.

8. System according to Claim 1, **characterized in that** the communication means (3) are configured to communicate using a communication network chosen among internet and GSM (4G/5G).

9. System according to Claim to any one of Claims 1 or 8, **characterized in that** the communication means (3) comprise a smartphone, a laptop or a digital pad.

10. System according to Claim 1, **characterized in that** the degrees of severity are chosen among severe, moderate and mild.

11. System according to Claim 1, **characterized in that** the graphical representation (6) comprises degrees of severity rated from 0 to 3.

12. System according to Claim 1, **characterized in that** the graphical representation (6) comprises the degrees of severity flagged by specific colors.

13. System according to Claim 5, **characterized in that** the graphical representation (6) comprises the general state of the patient flagged by a color.
